(19) Europäisches Patentamt
European Patent Office
Office européen des brevets

(11) Veröffentlichungsnummer: **0 175 647**
**A1**

(12) # EUROPÄISCHE PATENTANMELDUNG

(21) Anmeldenummer: **85810408.6**

(22) Anmeldetag: **09.09.85**

(51) Int. Cl.⁴: **D 21 D 3/00**, D 21 H 3/02, C 07 D 307/89

(30) Priorität: **13.09.84 CH 4390/84**

(43) Veröffentlichungstag der Anmeldung: **26.03.86 Patentblatt 86/13**

(84) Benannte Vertragsstaaten: **AT BE CH DE FR GB IT LI SE**

(71) Anmelder: **CIBA-GEIGY AG, Klybeckstrasse 141, CH-4002 Basel (CH)**

(72) Erfinder: **Bernheim, Michael, Dr., Sonnenweg 3, D-8901 Aystetten (DE)**
Erfinder: **Meindl, Hubert, Dr., Kornfeldstrasse 77, CH-4125 Riehen (CH)**
Erfinder: **Rohringer, Peter, Sechsjuchartenstrasse 1, CH-4124 Schönenbuch (CH)**

(54) **Verfahren zum Leimen von Papier oder Karton mit anionischen, hydrophoben Leimungsmitteln und kationischen Retentionsmitteln.**

(57) Anionische Leimungsmittel mit Anhydridgruppen, welche Verbindungen darstellen, die eine einzige N,N-$C_6$-$C_{22}$-Dialk(en)ylamidgruppe, N-$C_6$-$C_{22}$-Alk(en)ylamidgruppe oder $C_6$-$C_{22}$-Alk(en)ylestergruppe als hydrophoben Substituenten und mindestens eine anionische, acide oder in Salzform vorliegende Gruppe aufweisen, insbesondere das N,N-$C_6$-$C_{22}$-Dialk(en)ylamidocarbonsäure-anhydrid, N-$C_6$-$C_{22}$-Alk(en)ylamidocarbonsäure-anhydrid oder $C_6$-$C_{22}$-Alk(en)ylestercarbonsäure-anhydrid einer aromatischen Tetracarbonsäure oder deren Salze, eignen sich besonders gut dazu, zusammen mit handelsüblichen Retentionsmitteln in einem Verfahren zur Massenleimung oder Oberflächenleimung von Papier oder Karton eingesetzt zu werden.

EP 0 175 647 A1

CIBA-GEIGY AG
BASEL (Schweiz)

1-15068/+

# Verfahren zum Leimen von Papier oder Karton mit anionischen, hydrophoben Leimungsmitteln und kationischen Retentionsmitteln

Die vorliegende Erfindung betrifft ein Papierleimungsverfahren, bei welchem neben handelsüblichen, kationischen Retentionsmitteln ein Anhydridgruppen aufweisendes, aromatisches Leimungsmittel eingesetzt wird, das mindestens eine anionische oder saure Gruppe aufweist.

Aus z.B. der US Patentschrift 4 065 349 ist zwar auch ein Papierleimungsverfahren bekannt, bei welchem neben handelsüblichen, kationischen Retentionsmitteln ein Anhydridgruppen aufweisendes, aromatisches Leimungsmittel eingesetzt wird. Das in diesem bekannten Verfahren eingesetzte Leimungsmittel weist jedoch vorzugsweise keine anionische oder saure Gruppe auf im Gegensatz zum erfindungsgemäss eingesetzten Leimungsmittel, das zwingend eine solche anionische oder saure Gruppe aufweist. So wird im bekannten Verfahren insbesondere der Monoester einer als Anhydrid vorliegenden aromatischen Tricarbonsäure, z.B. der 4-n-Octadecylester von Trimellitsäureanhydrid, der keine saure Gruppe aufweist, als Leimungsmittel eingesetzt, während erfindungsgemäss insbesondere der Monoester einer als Anhydrid vorliegenden, aromatischen Tetracarbonsäure, z.B. der 4-n-Octadecylmonoester des Pyromellitsäureanhydrids, der eine saure Carboxylgruppe aufweist, als Leimungsmittel eingesetzt wird.

Es wurde nun gefunden, dass beim erfindungsgemässen Einsatz von aromatischen Leimungsmitteln, die neben Anhydridgruppen z.B. eine acide Carboxylgruppe aufweisen, zusammen mit handelsüblichen, kationischen Retentionsmitteln wesentlich bessere Leimungseffekte (gemessen z.B. an der Wasseraufnahme nach Cobb oder an der Tintenschwimmdauer) erzielt werden können als dies der Fall ist, wenn man neben handelsüblichen, kationischen Retentionsmitteln Anhydridgruppen aufweisende, Leimungsmittel einsetzt, die gemäss US Patentschrift 4 065 349 vorzugsweise keine acide Gruppe, insbesonders keine Carboxylgruppe aufweisen.

Gemäss vorliegender Erfindung werden dem Papierhersteller leicht zugängliche und auf einfache Art erhältliche Leimungsmittel zur Verfügung gestellt, die unter Mitverwendung von herkömmlichen, kationischen Retentionsmitteln in neuartiger Kombination geeignet sind, eine gute Leimung sowohl bei der Papierherstellung aus Faserstoffsuspensionen (Massenleimung) als auch bei der Herstellung von Papier mit geleimter Oberfläche (Oberflächenleimung) zu bewirken.

Der Gegenstand der vorliegenden Erfindung betrifft somit ein Verfahren zum Leimen von Papier oder Karton, d.h. ein Verfahren zur Herstellung von in der Masse geleimtem Papier oder Karton oder von Papier mit geleimter Oberfläche, das dadurch gekennzeichnet ist, dass man mindestens
(A) ein an sich neues, aromatisches Leimungsmittel, das eine Anhydridgruppe, eine einzige N,N-Dialkyl- oder N,N-Dialkenylamidgruppe, N-Alkyl- oder N-Alkenylamidgruppe oder Alkyl- oder Alkenylestergruppe mit jeweils mindestens 16 Kohlenstoffatomen im Alkyl- oder Alkenylrest als hydrophoben Substituenten und mindestens eine anionische, in Salzform vorliegende oder acide (saure), d.h. freie Gruppe aufweist und
(B) ein polymeres, kationisches Retentionsmittel
einsetzt.

Im erfindungsgemässen Verfahren werden bei der Massenleimung von Papier oder Karton die Komponenten (A) und (B) zu wässrigen, cellulosehaltigen, gegebenenfalls füllmittelhaltigen Faserstoffsuspensionen in beliebiger Reihenfolge oder gleichzeitig gegeben während bei der Papieroberflächenleimung das Papier mit einer wässrigen Leimflotte, welche die Komponenten (A) und (B) enthält, imprägniert und getrocknet wird.

Weitere Erfindungsgegenstände bilden

- die wässrigen Zusammensetzungen zur Durchführung des Papierleimungsverfahrens, die, sofern das Leimungsmittel (A) und das Retentionsmittel (B) bei der Massenleimung in beliebiger Reihenfolge zur Faserstoffsuspension separat gegeben werden, neben fakulatativen üblichen Zusätzen das Leimungsmittel (A) allein, das mindestens teilweise in Form von Salzen vorliegt, oder, sofern das Leimungsmittel (A) und das Retentionsmittel (B) bei der Massenleimung zur Faserstoffsuspension gleichzeitig gegeben werden, oder als Leimflotte zur Oberflächenleimung des Papiers eingesetzt werden, sowohl das gegebenenfalls mindesteens teilweise in Salzform vorliegende Leimungsmittel (A) als auch das Retentionsmittel (B) neben fakultativen, üblichen Zusätzen enthalten,
- nach dem erfindungsgemässen Verfahren geleimtes Papier oder geleimenter Karton,
- die Verwendung des Leimungsmittels (A) der angegebenen Art zum Leimen von Papier oder Karton und
- die als Leimungsmittel (A) eingesetzten, an sich neuen Verbindungen und das Verfahren zu deren Herstellung nach an sich bekannten Methoden.

Wie eingangs erklärt, weisen die erfindungsgemässen Leimungsmittel (A) als wesentliches Unterscheidungsmerkmal im allgemeinen mindestens eine anionische Gruppe auf, die in der Regel als acide Carboxyl-, Hydroxyl- oder Sulfogruppen vorliegt, wobei Sulfogruppen und vor allem Carboxylgruppen bevorzugt sind. Leimungsmittel, die zwei oder vor allem nur eine solche anionische Gruppe aufweisen, sind bevorzugt. Falls solche Gruppen als Salze, z.B. als Amin-, Ammonium-oder Natriumsalze vorliegen, können sie in wässrigem Medium bei den üblicherweise bei der Papierherstellung vorliegenden pH-Werten der Faserstoffsuspensionen Anionen bilden. Unter den genannten Bedingungen können andererseits auch die kationischen Retentionsmittel (B) Kationen bilden. Die Fähigkeit der Leimungsmittel und der Retentionsmittel, bei den Bedingungen der Papierherstellung Anionen bzw. Kationen zu bilden, kann auch als anionaktiv bzw. kationaktiv bezeichnet werden. Somit können die anionischen Leimungsmittel und die kationischen Retentionsmittel auch anionaktive Leimungsmittel bzw. kationaktive Retentionsmittel genannt werden.

Als weiteres kennzeichnendes Merkmal weisen die Leimungsmittel (A) als hydrophoben Substituenten eine einzige N,N-Dialkyl- oder N,N-Dialkenylamid-, N-Alkyl- oder N-Alkenylamid- oder Alkyl- oder Alkenylestergruppe auf. Die Alkyl- oder Alkenylreste in den angegebenen Amid- oder Estergruppen weisen unabhängig voneinander jeweils mindestens 6, vor allem 6 bis 22, vorzugsweise 11 bis 22, insbesondere 16 bis 20 Kohlenstoffatome auf. Alkylreste sind gegenüber Alkenylresten bevorzugt und leiten sich im allgemeinen von ungesättigten oder vor allem gesättigten, sekundären oder primären Fettaminen oder von ungesättigten oder vorzugsweise gesättigten Fettalkoholen ab. Hierbei sind symmetrische, sekundäre Amine, in welchen die beiden Alkenyl- oder Alkylreste identisch sind, gegenüber den asymmetrischen, sekundären Aminen mit zwei voneinander verschiedenen Alkyl- oder Alkenylresten bevorzugt. Die vorstehend erwähnten Fettamine und Fettalkohole leiten sich ihrerseits wiederum von ungesättigten oder vor allem gesättigten $C_6$-$C_{22}$-, vorzugsweise $C_{11}$-$C_{22}$-, insbesondere $C_{16}$-$C_{20}$-Fettsäuren ab. Bei diesen handelt es sich z.B. um Capron-, vorzugsweise Capryl-, Caprin-, Laurin-, Myristin- oder Myristolein-, Palmitolein-, Elaeostearin-, Clupanodonsäure, insbesondere Oel-, Elaidin-, Eruka-, Linol- und Linolensäure. Hierbei kommt der Palmitin-, Stearin-, Oel- und Behensäure eine besondere Bedeutung zu, wobei Palmitin- und vor allem Stearinsäure im Vordergrund des Interesses stehen. Auch gut zugängliche, technische Gemische dieser Fettsäuren kommen in Betracht. Synthetische Fettsäuren und Fettalkohole, die z.B. durch Oxosynthese herstellbar sind, werden von der angegebenen Definition auch umfasst.

Als bevorzugte, symmetrische, sekundäre Amine, aus welchen sich die N,N-Dialkyl- oder N,N-Dialkenylamidgruppen der erfindungsgemäss verwendeten Leimungsmittel ableiten, seien z.B. Dihexylamin,

Dioctylamin (auch Dicaprylamin genannt) und vor allem Didodecyl-, Dihexadecyl- und Dioctadecenylamin (auch Dilauryl-, Dipalmityl- und Dioleylamin genannt) erwähnt.

Seiner guten Zugänglichkeit wegen kommt dem Dioctadecylamin (auch Distearylamin genannt) eine besondere Bedeutung zu. Vor allem technische, handelsübliche Gemische der Fettamine der angegebenen Art kommen in Frage. So steht z.B. ARMEEN® 2 HT, das ein technisches Amingemisch mit einem mittleren Molekulargewicht von 500 darstellt, das als Hauptkomponente Distearylamin neben anderen, symmetrischen und asymmetrischen sekundären Aminen als Nebenkomponenten enthält, wobei diese Nebenkomponenten z.B. Oleyl-, Lauryl- und Palmitylreste aufweisen, im Vordergrund des Interesses.

Symmetrische und asymmetrische, sekundäre Amine der angegebenen Art sind aus den entsprechenden, vorstehend erwähnten Fettsäuren nach an sich bekannten Methoden erhältlich, indem die Fettsäure z.B. mit Ammoniak zum Nitril als Zwischenprodukt und anschliessend durch katalytische Hydrierung zum sekundären Amin umgesetzt wird.

Als spezifische Vertreter von primären Fettaminen, aus welchen sich die N-Alkyl- oder N-Alkenylamidgruppe der erfindungsgemäss verwendeten Leimungsmittel ableiten, seien z.B. Octylamin (auch Caprylamin genannt), vor allem Dodecyl-, Hexadecyl- und Octadecenylamin (auch Lauryl-, Palmityl- und Oleylamin genannt) und insbesondere seiner guten Zugänglichkeit wegen Octadecylamin (auch Stearylamin genannt) und als spezifische Vertreter von Fettalkoholen, aus welchem sich die Alkyl- oder Alkenylestergruppen des erfindungsgemäss verwendeten Leimungsmittel ableiten z.B. Hexadecanol, Oleylalkohol und seiner guten Zugänglichkeit wegen vor allem Octadecanol (auch Stearylalkohol genannt) erwähnt.

Vor allem auch technische Gemische der primären Fettamine oder der Fettalkohole der angegebenen Art kommen in Betracht.

Die als Komponente (A) erfindungsgemäss verwendeten Leimungsmittel, welche vorzugsweise eine einzige, gegebenenfalls als Salz vorliegende Carboxylgruppe als anionische Gruppe und eine einzige N,N-Dialkyl- oder N,N-Dialkenylamid-, N-Alkyl- oder N-Alkenylamid-oder Alkyl- oder Alkenylestergruppe der angegebenen Art aufweisen, stellen im allgemeinen das N,N-$C_6$-$C_{22}$-Dialkyl- oder -Dialkenylamidocarbonsäure-anhydrid, N-$C_6$-$C_{22}$-Alkyl- oder Alkenylamidocarbonsäure-anhydrid oder $C_6$-$C_{22}$-Alkyl- oder Alkenylestercarbonsäure-anhydrid einer aromatischen Tetracarbonsäure oder deren Salze dar, die sich vorzugsweise aus z.B. Naphthalintetracarbonsäure, vorzugsweise Benzophenontetracarbonsäure oder Pyromellitsäure als aromatische Tetracarbonsäure oder vor allem deren Dianhydride ableiten, die ihrer guten Zugänglichkeit wegen von besonderem Interesse sind. Somit stellen bevorzugte Leimungsmittel gegebenenfalls in Salzform vorliegende Verbindungen z.B. der Formel

(1) 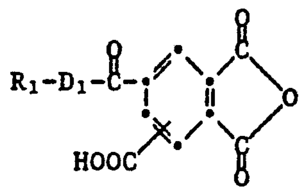

oder

(2) 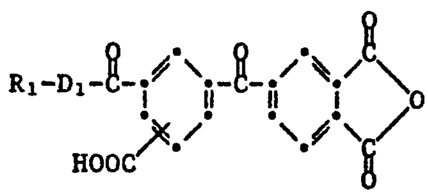

dar, worin

$R_1$ Alkyl oder Alkenyl mit 6 bis 22 Kohlenstoffatomen, $D_1$ -O-, -NH- oder -N($R_2$)- und $R_2$ die für $R_1$ angegebenen Bedeutungen hat und $R_1$ und $R_2$ voneinander verschieden oder vorzugsweise gleich sind, wobei die Leimungsmittel, welche gegebenenfalls in Salzform vorliegende Verbindungen der Formel

(3) 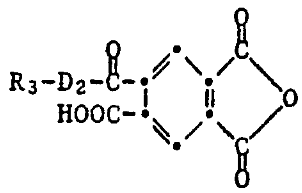

oder

(4) 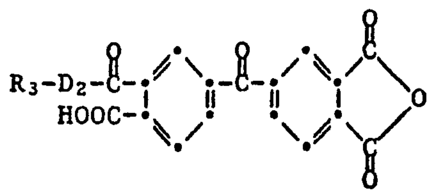

darstellen, worin $R_3$ Alkyl oder Alkenyl mit 16 bis 22 Kohlenstoffatomen und $D_2$ -O-, -NH- oder $-N(R_3)-$ bedeuten, im Vordergrund des Interesses stehen.

Als spezifische Vertreter der Leimungsmittel der Formel (3) oder (4) seien z.B. gegebenenfalls in Salzform vorliegende Verbindungen der Formeln

(5) 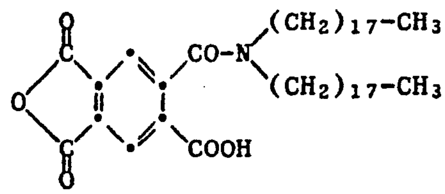 ,

(6) 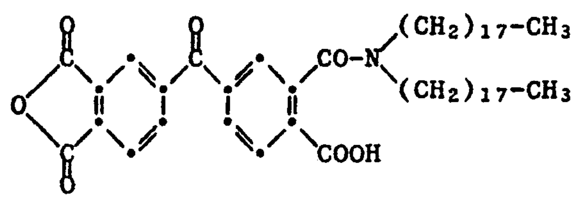 ,

(7) 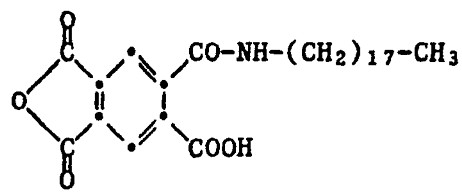 ,

(8) 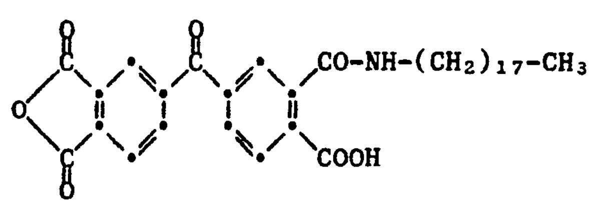 ,

(9) 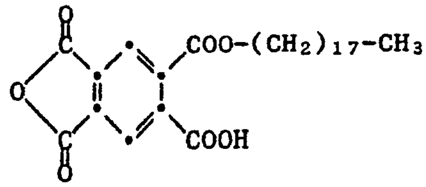

und

(10) 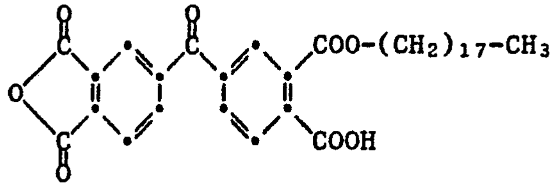

genannt.

Vor ihrem Einsatz als Komponente (A) im erfindungsgemässen Papierleimungsverfahren brauchen die Leimungsmittel nach erfolgter Herstellung im allgemeinen nicht durch z.B. Umkristallisieren gereinigt zu werden, sondern können in der Regel als solche, wie sie bei ihrer Herstellung anfallen, d.h. ohne weitere Aufarbeitung, verwendet werden.

Vor allem bei separater Zugabe (in beliebiger Reihenfolge) des Leimungsmittels (A) und des Retentionsmittels (B) zur Faserstoffsuspension beim erfindungsgemässen Verfahren zum Leimen von Papier oder Karton in der Masse ist es zweckmässig, das Leimungsmittel mindestens teilweise in Salzform einzusetzen. Solche Salze können bei Bedarf dadurch erhalten werden, dass man die Leimungsmittel (A) nach erfolgter Herstellung durch Zugabe u.a. eines Alkylamins oder Alkanolamins mit insgesamt höchstens 6 Kohlenstoffatomen, z.B. Trimethylamin, Triethylamin, Monoethanolamin, Diethanolamin, vor allem durch Zugabe von Ammoniak oder eines Alkalimetallhydroxides, beispielsweise Kalium- oder vor allem Natriumhydroxid, in der Regel in wässrigem Medium bei Raumtemperatur (etwa 15 bis etwa 25°C) in die entsprechenden Salze ganz oder teilweise überführt. Zweckmässigerweise wird ein Alkalimetallhydroxid, z.B. Kalium- oder vor allem Natriumhydroxid oder insbesondere Ammoniak in der Regel in Form

ihrer verdünnten, etwa 1 bis 10 gewichtsprozentigen, wässrigen Lösungen verwendet. Zweckmässig wird in der Regel höchstens 3 Mol, vor allem 0,1 bis 1,5, insbesondere 0,9 bis 1,1 Mol Ammoniak oder Alkalimetallhydroxid pro vorhandene acide Gruppe des Leimungsmittels eingesetzt. Die als Salze vorliegenden Leimungsmittel weisen somit z.B. acide Carboxyl-, Hydroxyl- oder Sulfogruppen auf, die mindestens teilweise in die Gruppen $-COO^{\ominus}M^{\oplus}$, $-O^{\ominus}M^{\oplus}$ oder $SO_3^{\ominus}M^{\oplus}$ überführt werden, worin $M^{\oplus}$ die entsprechenden Amin-, Ammonium- oder Alkalimetall-Kationen bedeutet.

Bevorzugte Leimungsmittel (A) der angegebenen Art weisen Molekulargewichte von etwa 300 bis etwa 1000, vorzugsweise etwa 450 bis etwa 900 auf.

Wie bereits angegeben, stellen die im erfindungsgemässen Papierleimungsverfahren als Komponente (A) eingesetzten Leimungsmittel an sich neue Verbindungen dar, die nach an sich bekannten Methoden hergestellt werden.

Somit sind insbesondere die gegebenenfalls in Salzform vorliegenden Verbindungen an sich neu, die vorzugsweise der Formel

(11) 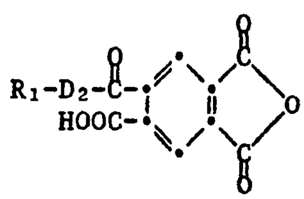

oder

(12) 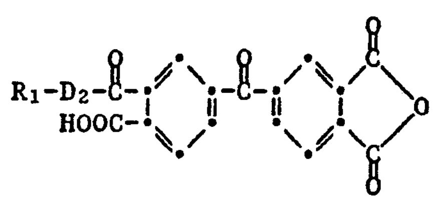

entsprechen, worin $D_2$ -O-, -NH- oder $-N(R_2)-$ und $R_1$ und $R_2$ voneinander verschieden oder vorzugsweise gleich sind und unabhängig voneinander jeweils Alkyl oder Alkenyl mit 6 bis 22, vorzugsweise 11

bis 22, insbesondere 16 bis 20 Kohlenstoffatomen bedeuten, wobei sich das Verfahren zu deren Herstellung dadurch auszeichnet, dass man einen Alkohol der Formel

(13) $R_1$-OH ,

ein primäres Amin der Formel

(14) $R_1$-$NH_2$

oder ein sekundäres Amin der Formel

(15) 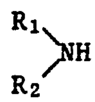 ,

worin $R_1$ und $R_2$ jeweils die angegebenen Bedeutungen haben, mit Pyromellitsäuredianhydrid oder Benzophenon-3,4,3',4'-tetracarbonsäuredianhydrid umsetzt, wobei vorzugsweise 1 Mol Fettalkohol der Formel (13), primäres Fettamin der Formel (14) oder sekundäres Fettamin der Formel (15) pro Mol Dianhydrid der angegebenen Art eingesetzt wird.

Im erfindungsgemässen Papierleimungsverfahren wird neben dem neuen, vorstehend beschriebenen, anionischen oder aciden Leimungsmittel (A) stets ein polymeres, kationisches Retentionsmittel (B) eingesetzt, welches in der Regel ein Molekulargewicht von mindestens etwa 1000, vorzugsweise etwa 2000 bis etwa 2'000'000 aufweist. Retentionsmittel mit Molekulargewichten im Bereich von 10'000 bis 100'000 sind besonders bevorzugt. Grundsätzlich kommt jedes handelsübliche Retentionsmittel für seinen Einsatz im erfindungsgemässen Verfahren in Betracht. Als Beispiele herkömmlicher Retentionsmittel (B), die sich besonders gut dazu eignen, zusammen mit dem Leimungsmittel (A) im erfindungsgemässen Papierleimungsverfahren eingesetzt zu werden, seien Polyalkylenimine; Epihalogenhydrin-Addukte von Umsetzungsprodukten aus Polyalkylenpolyaminen und aliphatischen Dicarbonsäuren; Epihalogenhydrin-Addukte von Umsetzungsprodukten aus Polyalkylenpolyaminen, Dicyandiamid und gegebenenfalls unveresterten oder mit Alkanolen veresterten, organischen Dicarbonsäuren; Umsetzungsprodukte aus Dicyandiamid, Formaldehyd, Ammoniumsalzen starker anorganischer Säuren und Alkylendiaminen oder Polyalkylenpolyaminen; kationisch modifizierte Stärken oder Kohlenhydrate aus Johannisbrot- oder Guarkernmehl; Copolymerisate auf Basis von Polyamid-Aminen und Umsetzungsprodukte aus Epihalogenhydrinen und polymerisierten Diallylaminen erwähnt.

Bevorzugte Epichlorhydrin-Addukte von Umsetzungsprodukten aus Polyalkylenpolyaminen und aliphatischen Dicarbonsäuren sind z.B. in der britischen Patentschrift 865 727, Epichlorhydrin-Addukte aus Umsetzungsprodukten aus Dicyandiamid und Diethylentriamin oder Triethylentetramin z.B. in der deutschen Offenlegungsschrift 2 710 061 und in der britischen Patentschrift 1 125 486, Epichlorhydrin-Addukte von Umsetzungsprodukten aus Diethylentriamin, Dicyandiamid und unveresterten oder vorzugsweise mit Niederalkanolen veresterten Dicarbonsäuren, insbesondere Dimethyladipat, z.B. in der britischen Patentschrift 1 125 486 und Umsetzungsprodukte aus Dicyandiamid, Formaldehyd, Ammoniumsalzen starker anorganischer Säuren und aus Ethylendiamin oder Triethylentetramin, z.B. in der US Patentschrift 3 491 064 beschrieben. Bevorzugte kationisch modifizierte Stärken oder Kohlehydrate aus Johannisbrot- oder Guarkernmehl sind z.B. Alkylenoxid-Addukte dieser Stärken oder Kohlenhydrate, wobei das eingesetzte Alkylenoxid 2 oder 3 Kohlenstoffatome im Alkylenrest und quaternäre Ammoniumgruppen aufweist oder insbesondere z.B. ein Trimethylglycidylammoniumhalogenid darstellt. Copolymerisate auf Basis von Polyamid-Aminen weisen Molekulargewichte von $10^3$ bis $10^5$, vorzugsweise $10^3$ bis $10^4$ auf und sind z.B. aus aliphatischen, gesättigten Dicarbonsäuren mit 2 bis 10, vorzugsweise 3 bis 6 Kohlenstoffatomen, insbesondere Adipinsäure, und Polyalkylenpolyaminen, z.B. Polypropylen- und Polyethylenpolyaminen, insbesondere Dimethylaminohydroxypropyl-diethylentriamin, erhältlich. Sie sind z.B. in CTFA Cosmetic Ingredient Dictionary, 3. Auflage 1982, der Cosmetic Toiletry and Fragrance Association beschrieben. Umsetzungsprodukte aus Epihalogenhydrinen

und polymerisierten Diallylaminen weisen bevorzugt Molekulargewichte von 1000 bis 2000 auf und sind z.B. in den US-Patentschriften 3 700 623 und 4 279 794 beschrieben.

Als Retentionsmittel (B), die zur Verwendung zusammen mit den Leimungsmitteln (A) im erfindungsgemässen Papierleimungsverfahren im Vordergrund des Interesses stehen, sei eine mit einem quaternäre Ammoniumgruppen enthaltenden Propylenoxyd modifizierte Mais- oder Kartoffelstärke, deren 25%ige Anschlämmung in destilliertem Wasser bei 20°C einen pH-Wert von 4,2 bis 4,6 aufweist, ein Polyethylenimin, das ein Molekulargewicht von 10'000 bis 100'000 aufweist, ein Epichlorhydrin-Addukt eines Umsetzungsproduktes aus Triethylentetramin und Dicyandiamid, ein Epichlorhydrin-Addukt eines Umsetzungsproduktes aus Diethylentriamin, Dicyandiamid und Dimethyladipat, ein Umsetzungsprodukt aus Dicyandiamid, Formaldehyd, Ammoniumchlorid und Ethylendiamin, ein Epichlorhydrin-Addukt eines Poly-N-methyldiallylamins und ein Copolymerisat aus Adipinsäure und Dimethylamino-hydroxypropyl-diethylentriamin genannt.

Verfahrensgemäss werden bei der Massenleimung von Papier oder Karton in der Regel 0,02 bis 3, vorzugsweise 0,05 bis 3, insbesondere 0,1 bis 0,8 Gewichtsprozent des Leimungsmittels (A) und 0,02 bis 3, vorzugsweise 0,05 bis 3, insbesondere 0,1 bis 0,4 Gewichtsprozent des Retentionsmittels (B), bezogen jeweils auf Trockensubstanz an (A) und (B) und auf den Feststoffgehalt der Faserstoffsuspension, eingesetzt. 0,02 bis etwa 0,05 Gewichtsprozent des Leimungsmittels (A) und des Retentionsmittels (B) reichen nur für das sogenannte "size press control", das mit konventionellen Leimungstests nicht erfassbar ist (vgl. z.B. Artikel "Control and Understanding of Size Press Pickup" von D.R. Dill in der Zeitschrift TAPPI (Proceedings of the Technical Association of the Pulp and Paper Industry, Band 57, Nr. 1 vom Januar 1974, Seiten 97-100). Die Faserstoffsuspension, zu welcher die Leimungsmittel (A) und die Retentionsmittel (B) gegeben werden, weist in der Regel einen Feststoffgehalt von 0,1 bis 5, vorzugsweise 0,3 bis 3, insbesondere 0,3 bis 1 Gewichtsprozent und einen Schopper-Riegler-Mahlgrad von etwa 10° bis etwa 60°, vor allem

20 bis 60°, vorzugsweise 20 bis 45°, insbesondere 25 bis 35°, auf. Sie enthält in der Regel Zellstoff, insbesondere solchen aus Nadelholz, z.B. Kiefernholz, oder aus Hartholz, d.h. Laubholz, z.B. Buchenholz, der nach herkömmlichen Verfahren, z.B. dem Sulfit- oder vor allem dem Sulfatverfahren hergestellt wird. Zudem enthält die Faserstoffsuspension gegebenenfalls Holzschliff. Auch alaunhaltiges Altpapier kann in der Faserstoffsuspension enthalten sein. Auch Zellstoffsuspensionen, die nach dem sogenannten CMP- oder CTMP-Verfahren (Chemimechanical and chemi-thermomechanical pulping processes, vgl. z.B. Artikel "Developments in Refiner Mechanical Pulping" von S.A. Collicutt und Mitarbeitern in TAPPI, Band 64, Nr. 6 vom Juni 1981, Seiten 57 bis 61) hergestellt werden, kommen in Betracht.

Die Faserstoffsuspension kann zudem organische oder mineralische Füllmittel enthalten. Als organische Füllmittel kommen u.a. synthetische Pigmente, z.B. Polykondensationsprodukte aus Harnstoff oder Melamin und Formaldehyd mit grossen spezifischen Oberflächen, die in hochdisperser Form vorliegen und z.B. in den britischen Patentschriften 1 043 937 und 1 318 244 beschrieben sind, als mineralische Füllmittel u.a. Montmorillonit, Titandioxid, Calciumsulfat und vor allem Talk, Kaolin und/oder Kreide (Calciumcarbonat) in Betracht. In der Regel enthält die Faserstoffsuspension 0 bis 40, vorzugsweise 5 bis 25, insbesondere 15 bis 20 Gewichtsprozent, bezogen auf den Feststoffgehalt der Faserstoffsuspension, an Trockensubstanz der Füllmittel der angegebenen Art.

Der pH-Wert der Faserstoffsuspension kann in einem weiten Bereich liegen, wobei z.B. Werte von 3,5 bis etwa 10 vorliegen können.

Bei Zusatz von z.B. Calciumcarbonat werden alkalische Faserstoffsuspensionen mit einem pH-Wert von etwa über 7 bis etwa 9, vorzugsweise 7,5 bis 8,5, erhalten. Saure Faserstoffsuspensionen mit einem pH-Wert von 3,5 bis 6,5 vorzugsweise 5 bis 6, können in Abwesenheit

von Calciumcarbonat durch Zugabe von Säuren, z.B. Schwefel- oder Ameisensäure oder vor allem von z.B. latent sauren Sulfaten, wie z.B. Aluminiumsulfat (Alaun), erhalten werden.

Faserstoffsuspensionen, die kein Füllmittel enthalten, können in einem breiten pH-Bereich von z.B. 3,5 bis 10 vorliegen. Bevorzugt sind Faserstoffsuspensionen, die gegebenenfalls durch Zusatz von Kreide einen pH-Wert von etwa 6,5 bis etwa 9, vorzugsweise 7 bis 9 aufweisen und insofern vorteilhaft sind, dass mögliche Korrosionserscheinungen an den empfindlichen Papiermaschinen ausgeschlossen werden. Zudem ist die Lagerfähigkeit von Papier oder Karton, das bei pH-Werten von 6,5 bis 9 der Faserstoffsuspension geleimt worden sind, gegenüber solchen, die bei pH-Werten von z.B. 5 bis 6 geleimt worden sind, deutlich überlegen.

Die Faserstoffsuspension kann auch Additive enthalten, wie z.B. Stärke oder ihre Abbauprodukte, welche die Faser/Faser- oder Faser/Füllmittel-Bindung erhöhen.

Auch hochmolekulare Polymere der Acrylsäurereihe, z.B. Polyacrylamide mit Molekulargewichten über 1'000'000, können zur Faserstoffsuspension als Hilfsmittel zum Zurückhalten feinster Zellstoff-Faserteilchen gegeben werden, wobei minimale Einsatzmengen von etwa 0,005 bis 0,02 Gewichtsprozent, bezogen auf Trockensubstanz des Polymers und den Feststoffgehalt der Faserstoffsuspensionen, genügend sind.

Die Faserstoffsuspension wird im erfindungsgemässen Massenleimungsverfahren auf an sich bekannte Weise auf Blattbildnern oder vorzugsweise kontinuierlich auf Papiermaschinen üblicher Bauart zu Papier oder Karton weiterverarbeitet. Nach einer Trocknung bei etwa 100 bis 140°C während etwa 0,5 bis 10 Minuten werden Papiere eines variablen Flächengewichtes von z.B. 50 bis 200 $g/m^2$ erhalten.

Zur Oberflächenleimung des Papiers im erfindungsgemässen Verfahren wird die Leimflotte, welche die Komponenten (A) und (B) enthält, z.B. durch Aufsprühen, vorzugsweise durch Foulardieren, in der Regel

bei Raumtemperatur (15-25°C) auf das Papier aufgebracht. Anschliessend wird das imprägnierte Papier bei 60 bis 140°C, vorzugsweise 90 bis 110°C während 0,1 bis 10, vorzugsweise 2 bis 6 Minuten getrocknet. Nach dem Trocknen wird ein Papier erhalten, das einen Flächenauftrag an Leimungs- und Retentionsmittel von 50 bis 150, vorzugweise 60 bis 120 $mg/m^2$ aufweist.

Bei dem erfindungsgemäss zu leimenden Papier handelt es sich um Papiere beliebiger Art mit beliebigen Flächengewichten, z.B. um Papier und Kartons aus gebleichter oder ungebleichter Sulfit- oder Sulfat-Cellulose.

Wie eingangs erwähnt, enthält die wässrige Zusammensetzung zur Durchführung des erfindungsgemässen Papierleimungsverfahrens neben fakultativen üblichen Zusätzen das Leimungsmittel (A), sofern das Leimungsmittel und das Retentionsmittel (B) bei der Massenleimung separat zur Faserstoffsuspension gegeben werden. In diesem Fall enthält die Zubereitung das Leimungsmittel in der Regel ganz oder vor allem teilweise in Form seiner Salze (erhalten unter Mitverwendung von z.B. Ammoniak, eines Alkyl- oder Alkanolamins oder eines Alkalimetallhydroxids der angegebenen Art in den vorstehend angegebenen Verhältnissen). Im allgemeinen enthalten solche Zusammensetzungen 5 bis 30, vorzugsweise 5 bis 20 Gewichtsprozent an Trockensubstanz des mindestens teilweise in Salzform vorliegenden Leimungsmittels, bezogen auf das Gesamtgewicht der wässrigen Zusammensetzung.

Hingegen enthält die wässrige Zusammensetzung neben den fakultativen, üblichen Zusätzen, sofern das Leimungsmittel (A) und das Retentionsmittel (B) bei der Massenleimung gleichzeitig zur Faserstoffsuspension gegeben werden,

(A) 2 bis 40, vorzugsweise 5 bis 30, insbesondere 5 bis 10 Gewichtsprozent Leimungsmittel (berechnet als Trockensubstanz), bezogen auf das Gesamtgewicht der wässrigen Zusammensetzung, wobei das Leimungsmittel gegebenenfalls in Salzform vorliegt, und

(B) 0,1 bis 20, vorzugsweise 0,5 bis 10, insbesondere 3 bis 8 Gewichtsprozent Retentionsmittel (berechnet als Trockensubstanz), bezogen auf das Gesamtgewicht der wässrigen Zusammensetzung.

Die wässrigen Zusammensetzungen der angegebenen Art enthalten gegebenenfalls als übliche Zusätze oberflächenaktive Verbindungen, z.B. Dispergatoren oder ferner Emulgatoren und/oder wasserlösliche, organische Lösungsmittel. Als Dispergatoren und Emulgatoren kommen z.B. herkömmliche Ligninsulfonate, Ligninсarboxylate, Carboxymethylcellulose, Ethylenoxidaddukte von Alkylphenolen, Fettaminen, Fettalkoholen oder Fettsäuren, Fettsäureester mehrwertiger Alkohole, substituierte Benzimidazole oder Kondensationsprodukte aus Formaldehyd und aromatischen Sulfonsäuren, vor allem Naphthalinsulfonsäuren in Betracht. Weitere oberflächenaktive Verbindungen sind vorzugsweise die anionischen Tenside, insbesondere Sulfattenside, z.B. Diethanolaminlaurylsulfat, Natriumlaurylsulfat oder ethoxylierte Laurylsulfate. Mögliche wasserlösliche, organische Lösungsmittel sind aliphatische Ether mit 1 bis 10 Kohlenstoffatomen, z.B. Dioxan, Ethylenglykol-n-butylether oder Diethylenglykolmonobutylether oder Alkohole mit 1 bis 4 Kohlenstoffatomen, z.B. Isopropanol, Ethanol oder Methanol.

Sofern die wässrigen Zusammensetzungen Zusätze der angegebenen Art enthalten, beträgt das Mengenverhältnis (Komponente (A)): (Zusätze) in der Zusammensetzung 1:0,02 bis 1:0,3, vorzugsweise 1:0,05 bis 1:0,1, bezogen auf Trockensubstanz der Leimungsmittel und der Zusätze.

Die Zusammensetzungen werden auf übliche Weise hergestelt, indem man das Leimungsmittel (A) zusammen mit dem Retentionsmittel (B) oder das Leimungsmittel (A) in der Regel teilweise in Form seines Salzes für sich allein entweder in geschmolzenem Zustand oder vorzugsweise in festem Zustand, insbesondere in pulverisierter Form, in der Regel in Gegenwart von Glasperlen und nötigenfalls von Emulgatoren (bei Leimungsmitteln in geschmolzenem Zustand) oder Dispergatoren (bei Leimungsmitteln in Pulverform) bei höchstens 90°C, vorzugsweise etwa

50 bis 85°C bei Emulsionen, insbesondere bei etwa 15 bis etwa 25°C bei Dispersionen, verrührt, wobei lagerstabile, homogene, weiterverdünnbare Emulsionen oder vorzugsweise Dispersionen erhalten werden. Da die Leimungsmittel zusammen mit den Retentionsmitteln oder die ganz oder mindestens teilweise als Salze vorliegenden Leimungsmittel in der Regel selbst-dispergierend oder selbst-emulgierend sind, ist der Einsatz von Dispergatoren oder Emulgatoren im allgemeinen nicht unbedingt erforderlich. Dies gilt auch für den fakultativen Zusatz von Lösungsmitteln und/oder Tensiden, die nur bei ungenügender Lagerstabilität der Dispersionen oder Emulsionen eingesetzt werden.

Bei der Oberflächenleimung von Papier wird die dazu benötigte Leimflotte durch Verdünnen mit Wasser der vorstehend angegebenen Emulsionen oder Dispersionen, die sowohl das Leimungsmittel (A) als auch das Retentionsmittel (B) enthalten, hergestellt. Hierbei werden die Emulsionen oder Dispersionen so verdünnt, dass eine Leimflotte entsteht, die (A) 0,02 bis 0,4 vorzugsweise 0,05 bis 3, insbesondere 0,05 bis 1 Gewichtsprozent Leimungsmittel (berechnet als Trockensubstanz), bezogen auf das Gesamtgewicht der wässrigen Leimflotte, wobei das Leimungsmittel gegebenenfalls in Salzform vorliegt, und (B) 0,01 bis 0,2, vorzugsweise 0,05 bis 0,1, insbesondere 0,3 bis 0,8 Gewichtsprozent Retentionsmittel (berechnet als Trockensubstanz), bezogen auf das Gesamtgewicht der wässrigen Leimflotte, enthält.

Als Vorteil des erfindungsgemässen Verfahrens sei erwähnt, dass in der Massenleimung Faserstoffsuspensionen der verschiedensten Art mit relativ besonders kleinen Mengen an Leimungs- und Retentionsmittel auf einfache Art und Weise zu Papier verarbeitet werden können, welche gute Leimungseigenschaften (Alkalitropfenprobe, Tintenschwimmdauer und vor allem Wasseraufnahme nach Cobb) aufweist. Dies gilt auch für die Oberflächenleimung, bei welcher die guten Leimungseffekte bereits mit geringen Flächenaufträgen an Leimungs-und Retentionsmittel erreicht werden. Insbesondere ermöglichen die geringen Flächenaufträge eine rasche Arbeitsweise, so dass bei der Trocknungstemperatur von z.B. 90 bis 110°C bereits innerhalb etwa 20 bis 40 Sekunden gute Oberflächenleimungen erzielt werden. Das

verfahrensgemäss in der Masse geleimte Papier weist gute mechanische Eigenschaften, d.h. gute Festigkeiten, insbesondere eine gute Reissfestigkeit auf. Eine gute Reproduzierbarkeit des Verfahrens sowohl bei der Massen- als auch bei der Oberflächenleimung ist gewährleistet. Insbesondere können bei der Massenleimung holzschliffhaltige oder altpapierhaltige Faserstoffsuspensionen verarbeitet werden. Auch die Kompatibilität des erfindungsgemäss verwendeten Leimungsmittels mit verschiedenen Füllmitteln wie z.B. Kaolin und auch verschiedenen Additiven, wie z.B. Alaun in sauerem Bereich der Faserstoffsuspensionen in der Massenleimung, ist vorteilhaft. Die erfindungsgemäss verwendeten Leimungs- und Retentionsmittel weisen eine gute Kompatibilität mit den üblichen, in der Papierindustrie verwendeten Hilfsstoffen wie Farbstoffen, Pigmenten, Bindemitteln, insbesondere optischen Aufhellern und sonstigen Zusatzstoffen auf. Im weiteren sind die eingesetzten Leimungs- und Retentionsmittel wie eingangs erwähnt leicht zugänglich und preisgünstig. Ferner neigen sie nicht zu einer unerwünschten Schaumbildung. Zudem wird der Weissgrad des geleimten Papiers durch die Leimung nicht wesentlich beeinflusst und kann sogar u.U. sowohl bei der Massen- als auch bei der Oberflächenleimung verbessert werden. Vor allem ist die in der Regel hohe Lagerstabilität der Leimungsmitteldispersionen der angegebenen Art von grossem Vorteil.

Die in den nachfolgenden Beispielen angegebenen Teile und Prozente beziehen sich auf das Gewicht.

<u>Herstellungsbeispiele neuer Verbindungen als Leimungsmitel</u>

<u>Beispiel 1</u>: 21,8 Teile Pyromellitsäuredianhydrid (0,1 Mol), 50,0 Teile eines technischen Distearylamingemisches (ARMEEN® 2 HT) mit einem mittleren Molekulargewicht von 500 (0,1 Mol) und 200 Teile Toluol werden auf die Rückflusstemperatur von ca. 111°C aufgeheizt und während 8 Stunden bei diesen Temperaturen unter Rühren gehalten, wobei allmählich eine klare Lösung entsteht. Das Lösungsmittel wird

anschliessend unter vermindertem Druck abdestilliert. Man erhält als gelbliche, zähflüssige Substanz 67,5 Teile der Verbindung, die im wesentlichen der Formel (5) entspricht.

Beispiel 2: Man verfährt wie in Beispiel 1 angegeben, setzt jedoch 32,2 Teile Benzophenontetracarbonsäuredianhydrid (0,1 Mol) (statt 21,8 Teilen Pyromellitsäuredianhydrid) ein. Man erhält als dickflüssige Substanz 78 Teile der Verbindung, die im wesentlichen der Formel (6) entspricht.

Beispiel 3: Zu einer Suspension von von 21,8 Teilen Pyromellitsäuredianhydrid (0,1 Mol) in 200 Teilen Chloroform wird bei 20° innerhalb von 15 Minuten eine Lösung von 26,8 Teilen Octadecylamin (0,1 Mol) in 150 Teilen Chloroform gegeben, wobei sich die Temperatur des Reaktionsgemisches von selbst auf 37°C erhöht. Danach wird das Reaktionsgemisch auf die Rückflusstemperatur von ca. 62°C weiter aufgeheizt und während 5 Stunden bei dieser Temperatur unter Rühren gehalten, wobei eine klare Lösung entsteht. Das Lösungsmittel wird anschliessend unter vermindertem Druck abdestilliert. Man erhält als weisses Pulver 46,5 Teile der Verbindung der Formel (7).
Schmelzpunkt: 143-149°C.

Beispiel 4: Man verfährt wie in Beispiel 3 angegeben, setzt jedoch 32,2 Teile Benzophenontetracarbonsäuredianhydrid (0,1 Mol) (statt 21,8 Teilen Pyromellitsäuredianhydrid) ein. Man erhält als weisses Pulver 29 Teile einer Verbindung, die der Formel (8) entspricht.
Schmelzpunkt: 145-151°C.

Beispiel 5: 10,9 Teile Pyromellitsäuredianhydrid (0,05 Mol) werden in 150 Teilen Dioxan gelöst und auf die Rückflusstemperatur von ca. 102°C aufgeheizt. Nun wird zu dieser Lösung innerhalb von 2 Stunden bei ca. 102°C eine Lösung von 13,5 Teilen Stearylalkohol (0,05 Mol) in 50 Teilen Dioxan gegeben. Das Reaktionsgemisch wird anschliessend während 20 Stunden bei der Rückflusstemperatur von ca. 102°C unter

Rühren gehalten. Das Lösungsmittel wird dann unter vermindertem Druck abdestiliert. Man erhält als weisses Pulver 23,0 Teile der Verbindung der Formel (9).
Schmelzpunkt: 135-138°C.

Beispiel 6: Man verfährt wie in Beispiel 5 angegeben, setzt jedoch 16,1 Teile Benzophenontetracarbonsäuredianhydrid (0,05 Mol) (statt 10,9 Teilen Pyromellitsäuredianhydrid) ein. Man erhält als gelbliche, zähflüssige Substanz 29,5 Teile der Verbindung der Formel (10).

Applikationsbeispiele

Beispiele 7 bis 10: Eine Faserstoffsuspension aus gebleichtem Birkensulfatzellstoff und Kiefernsulfatzellstoff im Gewichtsverhältnis 1:1 in Wasser von 10° dH (deutsche Härtegrade), die einen Schopper-Riegler-Mahlgrad von 35° und einen Feststoffgehalt von 0,5 % aufweist, wird mit 20 % Kreide als Füllmittel und hierauf mit 0,01 % PERCOL® 292 (kationisches, hochmolekulares (MG $>1 \cdot 10^7$) Polyacrylamid) als Hilfsmittel zum Zurückhalten feinster Zellstoffaserteilchen versetzt, wobei sich der in der nachfolgenden Tabelle I angegebene pH-Wert der Faserstoffsuspension einstellt. Die Prozentangaben beziehen sich auf Trockensubstanz an Hilfs- und Füllmittel, bezogen auf den Feststoffgehalt der Faserstoffsuspension.

Formulierungen des Leimungsmittels werden hergestellt, indem jeweils 7 % der angegebenen Leimungsmittel in Pulverform, wie sie bei der Herstellung anfallen, mit jeweils 3,5 % POLYMIN® P (Polyethylenimin mit einem Molekulargewicht von 10'000 bis 100'000) als Retentionsmittel in Gegenwart von entionisiertem Wasser und von Glasperlen mit einem Durchmesser von 2 mm bei Raumtemperatur (15 bis 25°C) verrührt werden. Die erhaltenen Dispersionen sind giessbar, homogen und lagerstabil. Die Prozentangaben geben den Trockensubstanzgehalt an Leimungs- und Retentionsmittel, bezogen auf das Gesamtgewicht der Formulierung an.

Nun wird die wässrige Formulierung des Leimungsmittels und des Retentionsmittels zur Faserstoffsuspension so gegeben, dass die in der Tabelle I angegebene Einsatzmenge an Trockensubstanz des Leimungsmittels, bezogen auf den Feststoffgehalt der Faserstoffsuspension, entsteht. Anschliessend wird die Faserstoffsuspension in einem Labor-Blattbildner "Formette Dynamique" der Fa. Allimand, Grenoble, Frankreich, zu Papierblättern verarbeitet, die nach der Trocknung bei 130°C während 3 Minuten ein Flächengewicht von 80 $g/m^2$ aufweisen.

Beide Oberflächen der erhaltenen Papierblätter, d.h. die auf der Siebseite des Blattbildners erhaltene Oberfläche und die Gegen- oder Oberseite werden auf ihre Leimungseigenschaften gesprüft. Zu diesem Zweck wird die Wasseraufnahme nach Cobb bei 30 Sekunden Einwirkungsdauer (WA $Cobb_{30}$) gemäss DIN 53 132 gemessen. Die Ergebnisse der WA $Cobb_{30}$-Messungen in $g/m^2$ der Siebseite (SS) und Oberseite (OS) nach der Trocknung bei 130°C und nach einer Lagerung von einem Tag bei 23°C und einer relativer Feuchtigkeit von 50 % sind in der nachfolgenden Tabelle I angegeben. Je geringer die Wasseraufnahme, desto besser ist die Leimung des Papiers. WA $Cobb_{30}$ Werte über 100 entsprechen einer völlig unbefriedigenden Leimung des Papiers.

Tabelle I

| Beispiel Nr. | Leimungsmittel | Einsatzmenge Leimungsmittel % | pH-Wert der Faserstoff Suspension | WA $Cobb_{30}$ ($g/m^2$) | | | |
|---|---|---|---|---|---|---|---|
| | | | | nach Trocknung | | nach 1 Tag Lagerung | |
| | | | | SS | OS | SS | OS |
| 7 | Verbindung gemäss Beispiel 1 | 1 | 8,3 | 28 | 17 | 20 | 14 |
| 8 | Verbindung gemäss Beispiel 2 | 2 | 8,1 | 13 | 18 | 11 | 13 |
| 9 | Verbindung gemäss Beispiel 3 | 2 | 8,5 | 33 | 17 | — | — |
| 10 | Verbindung gemäss Beispiel 4 | 2 | 8,1 | 32 | 15 | 31 | 14 |

Aehnliche Ergebnisse werden erzielt, wenn man anstelle von POLYMIN® P als Retentionsmittel CATO® 110 (kationisch modifizierte Stärke, die mit einem quaternären Ammoniumgruppen enthaltenden Propylenoxid modifiziert ist und deren pH-Wert der 25%igen Anschlämmung in destilliertem Wasser bei 20°C 4,2 bis 4,6 beträgt), POSAMYL® E7 (kationisch modifizierte Stärke mit einem Stickstoffgehalt von 0,4 %), einer mit Trimethylglycidylammoniumchlorid kationisch modifizierte, native Kartoffelstärke, deren Stickstoffgehalt 1,3 % beträgt, ein Kondensationsprodukt aus Dicyandiamid und Triethylentetramin, das mit Epichlorhydrin weiter umgesetzt und z.B. gemäss Beispiel 2 der deutschen Offenlegungsschrift 2 710 061 hergestellt wird, ein Epichlorhydrinaddukt eines Umsetzungsproduktes aus Diethylendiamin und Adipinsäure, das z.B. gemäss Beispiel 1 der britischen Patentschrift 865 727 hergestellt wird, ein Umsetzungsprodukt aus Dicyandiamid, Formaldehyd, Ammoniumchlorid und Ethylendiamin, das z.B. gemäss Beispiel 1 der US-Patentschrift 3 491 064 hergestellt wird, oder RETAMINOL® K (Polyethylenimin eines Molekulargewichtes von 20'000 bis 40'000) einsetzt. Auch Gemische der Retentionsmittel der vorstehend angegebenen Art kommen hierbei in Betracht. Zur Erzielung guter Ergebnisse ist nötigenfalls ein Zusatz von Dispergatoren, insbesondere von Kondensationsprodukten aus Formaldehyd und Naphthalinsulfonsäuren oder von Carboxymethylcellulose vorteilhaft. Hingegen wird nur eine schlechte Leimung mit Cobb-Werten von etwa 150 bis etwa 200 erhalten, wenn man ein Leimungsmittel gemäss einem der Beispiele 1 bis 4, jedoch kein Retentionsmittel, oder ein Retentionsmittel der vorstehend angegebenen Art, jedoch kein Leimungsmittel einsetzt.

Beispiele 11 bis 16: Man verfährt wie in Beipielen 7 bis 10 angegeben, gibt jedoch das Leimungsmittel und das Retentionsmitel separat zur Faserstoffsuspension, wobei 7, 10 oder 15 % Leimungsmittel in Pulverform bei Raumtemperatur (15 bis 25°C) in Gegenwart von Wasser und Glasperlen mit einer wässrigen, 5%igen Ammoniaklösung zu einer selbstemulgierenden, ebenfalls giessbaren, homogenen und lagerstabilen Emulsion verrührt werden und wobei die in der nachfolgenden

Tabelle II angegebenen Formulierungen des Leimungsmittels entstehen. Die angegebenen Val% bedeuten die Anzahl Aequivalente an Ammoniak für 100 Aequivalente, bezogen auf die Anzahl vorhandener, acider Gruppen der jeweils eingesetzten Leimungsmittel. 10 Sekunden nach der Zugabe der angegebenen Einsatzmenge an Trockensubstanz POLYMIN® P als Retentionsmittel wird die Faserstoffsuspension mit jeweils der angegebenen Einsatzmenge an Trockensubstanz des Leimungsmittels versetzt, wobei sich die Einsatzmenge an Leimungs- und Retentionsmittel auf den Feststoffgehalt der Faserstoffsuspension beziehen. Die Leimungsergebnisse sind ebenfalls aus der Tabelle II zu entnehmen.

Tabelle II

| Beispiel Nr. | Leimungsmittel Formulierung | Einsatzmenge Leimungsmittel (%) | Einsatzmenge Retentionsmittel (%) | pH-Wert der Faserstoff-Suspension | WA $Cobb_{30}$ ($g/m^2$) nach Trocknung SS | OS |
|---|---|---|---|---|---|---|
| 11 | 15 % Verbindung gemäss Beispiel 1 100 Val% Ammoniak | 0,50 | 0,25 | 8,6 | 19 | 14 |
| 12 | 15 % Verbindung gemäss Beispiel 2 100 Val% Ammoniak | 0,50 | 0,25 | 8,7 | 20 | 16 |
| 13 | 10 % Verbindung gemäss Beispiel 3 100 Val% Ammoniak | 1,0 | 0,75 | 8,5 | 60 | 19 |
| 14 | 10 % Verbindung gemäss Beispiel 4 100 Val% Ammoniak | 0,50 | 0,25 | 8,4 | 23 | 14 |
| 15 | 10 % Verbindung gemäss Beispiel 5 100 Val% Ammoniak | 1,0 | 0,75 | 8,6 | 25 | 20 |
| 16 | 10 % Verbindung gemäss Beispiel 6 100 Val% Ammoniak | 1,0 | 0,75 | 8,5 | 27 | 17 |

Bei Verwendung von 10 bis 300 Val% Ammoniak oder Natriumhydroxid (als 5%ige wässrige Lösung) zur Formulierung des Leimungsmittels werden ähnlich gute Leimungsergebnisse wie die in Tabelle II angegebenen erhalten.

Aehnliche Ergebnisse werden auch erzielt, wenn man zur Faserstoffsuspension das Leimungsmittel zuerst und 10 Sekunden hierauf das Retentionsmittel gibt. Das gleiche gilt auch, wenn auf die Zugabe von PERCOL® 292 und/oder auf die Zugabe eines Füllmittels verzichtet wird. Aehnliche Ergebnisse werden ebenfalls erzielt, wenn man anstelle von Kreide als Füllmittel Talk oder Kaolin oder wenn man zusätzlich Alaun einsetzt. Auch bei Einsatz von holzschliffhaltigen Faserstoffsuspensionen werden gute Leimungsergebnisse erhalten.

Patentansprüche

1. Verfahren zum Leimen von Papier oder Karton, dadurch gekennzeichnet, dass man mindestens
(A) ein aromatisches Leimungsmittel, das eine Anhydridgruppe, eine einzige N,N-Dialkyl- oder N,N-Dialkenylamidgruppe, N-Alkyl- oder N-Alkenylamidgruppe oder Alkyl- oder Alkenylestergruppe mit je mindestens 6 Kohlenstoffatomen im Alkyl- oder Alkenylrest als hydrophoben Substituenten und mindestens eine anionische, acide oder in Salzform vorliegende Gruppe aufweist und
(B) ein polymeres, kationisches Retentionsmittel,
einsetzt.

2. Verfahren nach Anspruch 1, dadurch gekennzeichnet, dass man zur Massenleimung von Papier oder Karton die Komponenten (A) und (B) zu wässrigen, cellulosehaltigen, gegebenenfalls füllmittelhaltigen Faserstoffsuspensionen in beliebiger Reihenfolge oder gleichzeitig gibt.

3. Verfahren nach Anspruch 1, dadurch gekennzeichnet, dass man zur Papieroberflächenleiumung das Papier mit einer wässrigen Leimflotte, welche die Komponenten (A) und (B) enthält, imprägniert und trocknet.

4. Verfahren nach einem der Ansprüche 1 bis 3, dadruch gekennzeichnet, dass man als Komponente (A) das N,N-$C_6$-$C_{22}$-Dialkyl oder -Dialkenylamidocarbonsäure-anhydrid, das N-$C_6$-$C_{22}$-Alkyl- oder -Alkenylamidocarbonsäure-anhydrid oder das $C_6$-$C_{22}$-Alkyl- oder -Alkenylester-carbonsäure-anhydrid einer aromatischen Tetracarbonsäure oder deren Salze einsetzt.

5. Verfahren nach Anspruch 4, dadurch gekennzeichnet, dass man als Komponente (A) Leimungsmittel einsetzt, die gegebenenfalls in Salzform vorliegende Verbindungen der Formel

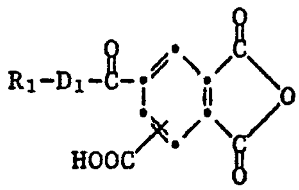

oder

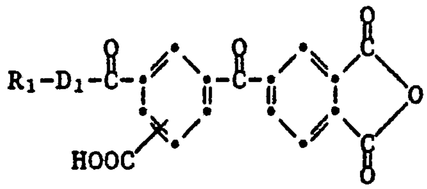

darstellen, worin

$R_1$ Alkyl oder Alkenyl mit 6 bis 22 Kohlenstoffatomen, $D_1$ -O-, -NH- oder $-N(R_2)-$ und $R_2$ die für $R_1$ angegebenen Bedeutungen hat und $R_1$ und $R_2$ gleich oder voneinander verschieden sind.

6. Verfahren nach einem der Ansprüche 1 bis 5, dadurch gekennzeichnet, dass man als Retentionsmittel (B) ein Polyalkylenimin; Epihalogenhydrin-Addukte von Umsetzungsprodukten aus Polyalkylenpolyaminen und aliphatischen Dicarbonsäuren oder von Umsetzungsprodukten aus Polyalkylenpolyaminen, Dicyandiamid und gegebenenfalls unveresterten oder mit Alkanolen veresterten, organischen Dicarbonsäuren; Umsetzungsprodukte aus Dicyandiamid, Formaldehyd, Ammoniumsalzen starker anorganischer Säuren und Alkylendiaminen oder Polyalkylenpolyaminen; kationisch modifizierte Stärken oder Kohlenhydrate aus Johannisbrot-oder Guarkernmehl; Copolymerisate auf Basis von Polyamid-Aminen; oder Umsetzungsprodukte aus Epihalogenhydrinen und polymerisierten Diallylaminen einsetzt.

7. Wässrige Zusammensetzung zur Durchführung des Verfahrens gemäss Anspruch 2, wobei das Leimungsmittel (A) und das Retentionsmittel (B) in beliebiger Reihenfolge separat zur Faserstoffsuspension

gegeben werden, dadurch gekennzeichnet, dass sie das Leimungsmittel (A), mindestes teilweise in Form von Salzen, und gegebenenfalls übliche Zusätze enthält.

8. Wässrige Zusammensetzung zur Durchführung des Verfahrens gemäss Anspruch 2, wobei das Leimungsmittel (A) und das Retentionsmittel (B) gleichzeitig zur Faserstoffsuspension gegeben werden, dadurch gekennzeichnet, dass sie
(A) 2 bis 40 Gewichtsprozent Leimungsmittel und
(B) 0,1 bis 20 Gewichtsprozent Retentionsmittel,
bezogen jeweils auf Trockensubstanz von (A) und (B) und auf das Gesamtgewicht der wässrigen Zusammensetzung, und gegebenenfalls übliche Zusätze enthält.

9. Wässrige Leimflotte als Zusammensetzung zur Durchführung des Verfahrens gemäss Anspruch 3, dadurch gekennzeichnet, dass sie
(A) 0,2 bis 0,4 Gewichtsprozent Leimungsmittel und
(B) 0,01 bis 0,2 Gewichtsprozent Retentionsmittel,
bezogen jeweils auf Trockensubstanz von (A) und (B) und auf das Gesamtgewicht der wässrigen Zusammensetzung, und gegebenenfalls übliche Zusätze enthält.

10. Nach dem Verfahren gemäss einem der Ansprüche 1 bis 6 geleimtes Papier oder geleimter Karton.

11. Verwendung der Komponente (A) gemäss einem der Ansprüche 1 bis 5 zum Leimen von Papier oder Karton.

12. Gegebenenfalls in Salzform vorliegende Verbindungen der Formel

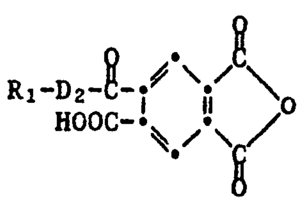

oder

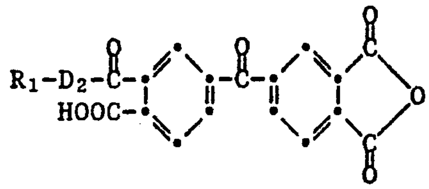

worin $D_2$ -O-, -NH- oder $-N(R_2)-$ und $R_1$ und $R_2$ je Alkyl oder Alkenyl mit 6 bis 22 Kohlenstoffatomen bedeuten.

13. Verfahren zur Herstellung der Verbindungen gemäss Anspruch 12, dadurch gekennzeichnet, dass man einen Alkohol der Formel

$$R_1\text{-}OH \quad ,$$

ein primäres Amin der Formel

$$R_1\text{-}NH_2$$

oder ein sekundäres Amin der Formel

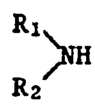 ,

worin $R_1$ und $R_2$ je Alkyl oder Alkenyl mit 6 bis 22 Kohlenstoffatomen bedeuten, mit Pyromellitsäuredianhydrid oder Benzophenon-3,4,3',4'-tetracarbonsäuredianhydrid umsetzt.

FO 7.1 PLP/eg*/cs*

0175647

Europäisches Patentamt

**EUROPÄISCHER RECHERCHENBERICHT**

Nummer der Anmeldung

EP 85 81 0408

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| D,X | US-A-4 065 349 (M.E. BATEMAN u.a.)<br>* Insgesamt und insbesondere Anspruch 13 * | 1,2,4-8,10-12 | D 21 D 3/00<br>D 21 H 3/02<br>C 07 D 307/89 |
| D,A | US-A-4 279 794 (D.H. DUMAS)<br>* Insgesamt * | 1-3,6-11 | |
| A | CHEMICAL ABSTRACTS, Band 90, Nr. 11, 12. März 1979, Seite 577, Nr. 86958m, Columbus, Ohio, US; W.I. AWAD u.a.: "Dehydration of pyromellitamic acids", & J. IRAQI. CHEM. SOC. 1977, 2(2), 39-51<br>* Insgesamt * | 12 | |
| | | | RECHERCHIERTE SACHGEBIETE (Int Cl 4) |
| A | FR-A-2 338 272 (CIBA-GEIGY)<br>* Seiten 31,32, Beispiele 8,9 * | 12,13 | C 07 D<br>D 21 H |

Der vorliegende Recherchenbericht wurde für alle Patentansprüche erstellt.

| Recherchenort | Abschlußdatum der Recherche | Prüfer |
|---|---|---|
| DEN HAAG | 13-12-1985 | NESTBY K. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82